# EUROPEAN PATENT SPECIFICATION

(11) **EP 1 878 390 B1**
(45) Date of publication and mention of the grant of the patent: **01.04.2009**
(21) Application number: 06014766.7
(22) Date of filing: 14.07.2006
(51) Int. Cl.: A61B 17/02, A61B 17/122

(54) **Surgical retractor**
Aufblasbarer, chirurgischer Retraktor
Ecarteur chirurgical gonflable

(43) Date of publication of application: 16.01.2008
(73) Proprietor: Surgery in Motion Ltd., 13 Kensington Square London W8 5HD (GB)
(72) Inventor: Mehmanesh, Hormoz, Tehran (IR)
(74) Representative: Grünecker, Kinkeldey, Stockmair & Schwanhäusser Anwaltssozietät

(56) References cited:
- EP-A- 1 230 899
- WO-A-99/15082
- DE-A- 19 947 885
- US-A- 5 236 437
- US-A- 5 342 385
- US-A- 5 454 826
- US-A- 5 827 289
- US-A- 6 036 706
- US-A1- 2003 181 939
- US-A1- 2004 002 726

## Description

The present invention generally relates to surgical instruments, more specifically to a surgical retractor comprising at least one inflatable element being adapted to exhibit a force on a part of a human or animal body upon inflation. Surgical retractors are used in surgery to retract organs or tissue in order to gain exposure of the operative site. Surgical retractors comprise, inter alia, sternotomy retractors which are used in surgery of the thorax, in particular in heart surgery. A sternotomy retractor according to the state of the art comprises a stationary arm and a movable arm. A cross bar is attached to one end of the stationary arm. An end of the movable arm is movably engaged with the cross-bar. Thus, the sternotomy retractor has a shape similar to the letter "U". On the stationary and the movable arm, jaws which are adapted to engage portions of a sternum are provided. Typically, surgical retractors according to the state of the art are made of stainless steel.

In surgery, an incision beginning at the jugular region of the patient and cutting medially the entire length of the sternum up to the xiphoid area is made. Then, the entire length of the sternum or, alternatively, a portion of the sternum only, is split, for example by means of an electric saw. Thereafter, the sternotomy retractor, with the movable arm being located besides the stationary arm, is inserted into the split sternum such that the jaws of the fixed and the movable arm each engage one of the portions of the sternum and the movable arm is moved away from the stationary arm. Thus, the sternum and the rib cage of the patient are forced to separate and expand open, giving access to the mediastinum of the patient. After the surgery, the sternotomy retractor is removed, and the thorax of the patient is closed. Thereby, an osteosynthesis of the sternum is performed, for example by means of stainless steel wires and/or a sternum tape.

Since the rib cage of the patient may have a considerable mechanical rigidity, a relatively large force may be required in order to open the thorax of a patient, in particular, if the patient is large and obese or has a strong sternum and rib cage area. If a sternotomy retractor according to the state of the art made of stainless steel is used, due to the inelasticity of the retractor, considerable mechanical strain may occur in parts of the sternum, which may lead to damage or even fracture of the bone tissue, especially in patients with osteoporotic bones.

A further type of surgical retractors known to persons skilled in the art comprises aortic clamps. In some surgical operations performed at the open heart of a patient, the patient is connected to a heart-lung machine taking over the functions of the patient's heart and lung during surgery. Thereafter, the patient's aorta is clamped off by means of a conventional clamp of a type known to persons skilled in the art as "aortic clamp" and the patient's heartbeat is stopped by inserting a cardioplegic solution. After surgery, the aortic clamp is removed and the patient's heartbeat is restored.

Surgical retractors are also used in the implantation of artificial heart valves. In the insertion of an artificial heart valve, an access to the heart valve is created, for example by providing a cut in the patient's aorta in case of a replacement of the aortic valve, or by providing a cut in the left atrium wall in case of a replacement of the mitral valve. Thereafter, a retractor known to persons skilled in the art as aortic retractor such as "Langenbeck retractor" is inserted into the aorta or a mitral clamp in the left atrium, respectively, in order to retract the tissue of the aorta and/or the atrium and expose the aortic or mitral valve.

Aortic clamps and surgical retractors for use in heart valve surgery known to persons skilled in the art are made from hard, inelastic materials such as stainless steel. Therefore, the sensitive tissue of the heart and/or the aorta may easily be damaged during surgery.

It is, therefore, a problem of surgical retractors known to persons skilled in the art that tissue may easily be damaged during surgery. Moreover, the shape of surgical retractors according to the state of the art may not be adapted to the individual anatomy of the patient. Thus it is very difficult to adjust to individual variations of size and shape of the anatomy by using inflexible instruments.

WO 99/15082 discloses a surgical support apparatus which lifts and supports a patient's heart or other organ in a steady position during surgery. The apparatus includes a handle releasably connected to a plate on which an expandable membrane and at least one conduit connected to the expandable membrane are provided. The expandable membrane is filled with an inflation fluid to lift and support the organ. The conduit is securely attached to the plate or membrane, so that if the handle is removed, the conduit may be used to remove the plate from the patient by pulling the conduit. The two part form of the present independent claim is based on this prior art.

A problem of surgical retractors according to the state of the art is that handles are required in order to operate the retractors. When the retractor is put in place, such handles may obstruct the operation field of the surgeon. Moreover, it is necessary that the retractor is held in place by surgical assistants.

It is an object of the present invention to provide a surgical retractor which allows to reduce a risk of tissue being damaged when the retractor is used. A further object is to provide a surgical retractor which may adjust to the individual anatomic variations of the patients.

According to the present invention, these objects are achieved by providing a surgical retractor comprising a rigid holder, wherein at least one inflatable element is connected to the rigid holder and is adapted to exhibit a force on a part of a human or animal body upon inflation.

The rigid holder comprises a fluid line adapted to supply fluid to the at least one inflatable element and/or to discharge fluid from the at least one inflatable element. The rigid holder comprises a first portion and a second portion detachably connected with each other. The second portion is connected to the inflatable element. The fluid line extends through the first portion and the second portion. The second portion comprises a valve closeable on detachment of the first portion.

When a force is exhibited on the part of the body by means of the inflatable element, the inflatable element may deform and, thus, adapt to the individual anatomy of the patient. Thus, the force may be more uniformly applied and the occurrence of high local strain which may damage the patient's tissue can be avoided. Moreover, by varying the pressure in the inflatable element, a degree of hardness of the surgical retractor may be adapted to the specific requirements of surgery. A relatively hard surgical retractor may be obtained by providing a high pressure in the inflatable element, whereas a softer surgical retractor can be obtained by providing a low pressure in the inflatable element. Moreover, the size of the retractor may be varied by controlling the pressure in the inflatable element. While the inflatable element can be small if no pressure or low pressure is applied, the size of the inflatable element can be increased by inserting a fluid, for example filtered air or CO₂ under pressure in order to inflate the inflatable element. This may help inserting the retractor into small openings such as, for example, incisions in blood vessels or heart chambers.

Providing a rigid holder comprising a fluid line adapted to supply fluid to the at least one inflatable element and/or to discharge fluid from the at least one inflatable element allows to avoid an obstruction of the operational field by separate fluid lines. In surgery, the surgical retractor can be inserted into the patient's body and the at least one inflatable element can be inflated in order to gain exposure of the operative site. Then, the first portion of the rigid holder can be removed in order to reduce an obstruction of the operational field. Moreover, since the second portion of the rigid holder has a lower weight than the entire rigid holder, a moment of force imparted to the at least one inflatable element by the weight of the rigid holder can be reduced. Thus, the inflatable element may remain in place without there being a necessity to hold the rigid holder by hand.

In embodiments of the present invention, the inflatable element comprises a material having an anisotropic elasticity. Thus, the variation of the size of the inflatable element upon insertion of a fluid such as air or CO₂ under pressure may be directional.

In some embodiments, the material having an anisotropic elasticity can be extensible in one direction and non-extensible in another direction. Thus, an extension of the inflatable element in a dimension corresponding to the non-extensible direction may be kept constant, while the extension of the inflatable element in a dimension corresponding to the extensible direction can be controlled by varying the pressure in the inflatable element.

The inflatable element can comprise an elastic envelope and at least one spacer element adapted to maintain a predetermined distance between two portions of the elastic element. By providing the at least one spacer element, the anisotropy of the elasticity of the elastic envelope can be controlled.

In some embodiments of the present invention, the two portions of the elastic envelope connected by the spacer elements are provided on opposite inner surfaces of an inflatable volume of the inflatable element. Thus, a diameter of the inflatable volume, measured in the direction of the at least one spacer element, may be kept substantially constant upon inflation of the inflatable element.

The spacer element may comprise a chordal bridge. Thus, the predetermined distance between the portions of the envelope connected by the spacer element may reliably be maintained.

The surgical retractor can be configured as a heart valve retractor.

In a heart valve retractor, the at least one inflatable element may have a toroidal shape comprising an inner circumferential surface and an outer circumferential surface. Moreover, the inflatable element can comprise a plurality of spacer elements provided inside the inflatable element and being adapted to maintain a predetermined distance between the inner circumferential surface and the outer circumferential surface. Thus, the retractor can be inserted into the patient's aorta or other heart chambers with no pressure at all or low pressure being applied to the inflatable element. Thereafter, the inflatable element is inflated to increase the radius of the toroid. Thus, the inflatable element is applied to the aorta or atrium, respectively. The presence of the spacer elements avoiding an increase of the thickness of the inflatable element which might lead to an undesirable obstruction of the operational field. In this way the transsection of the balloon keeps its fixed diameter while the inner and outer diameter of the balloon increase synchronically.

In further embodiments of the present invention, the surgical retractor can be configured as a blood vessel clamp.

In a blood vessel clamp, the rigid holder can comprise a first clamp portion and a second clamp portion. A blood vessel is insertable between the first clamp portion and the second clamp portion. An inflatable element is provided on each of the first clamp portion and the second clamp portion. Thus, the inflatable element allows to prevent a contact between the rigid holder and the blood vessels, which allows reducing a risk that the blood vessel is damaged by the clamp portions. Moreover, a pressure exhibited on the blood vessel can be precisely controlled by varying the pressure in the inflatable elements.

Embodiments of the present invention will now be described with reference to the accompanying drawings, wherein
- Fig. 1: shows a schematic perspective view of a heart valve retractor according to an embodiment of the present invention;
- Figs. 2a - 2c: show a schematic perspective views of portions of the heart valve retractor shown in Fig. 1;
- Fig. 3: shows a schematic cross-sectional view of an inflatable element of the heart valve retractor shown in Figs. 1 and 2;
- Fig. 4: shows a schematic perspective view of a blood vessel clamp according to an embodiment of the present invention;
- Figs. 5a and 5b: show further schematic perspective views of the blood vessel clamp shown in Fig. 4;

Although the following description focuses on retractor systems in the field of heart surgery, the present invention may be applied to all fields of surgical subspecialties, like urology, obstetrics and gynecology, neurosurgery or general surgery even and specifically in minimally invasive and endoscopic surgery.

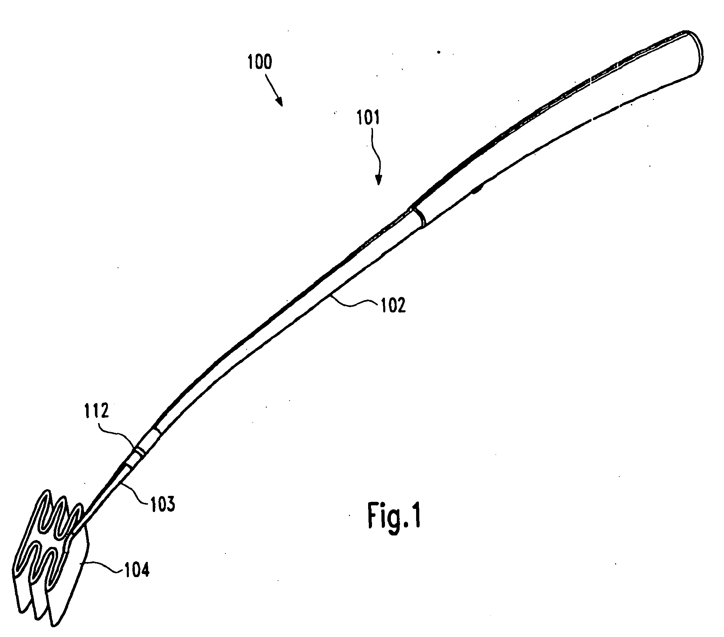

Fig. 1 shows a schematic perspective view of a heart valve retractor 100 according to an embodiment of the present invention. The heart valve retractor 100 comprises a rigid holder 101. The rigid holder 101 comprises a first portion 102 and a second portion 103. The second portion 103 is detachably connected to the first portion 102 via a connection element 112. In the following, the term "rigid holder" shall include components being formed from a substantially inelastic material, for example plastics, ceramics or stainless steel. As will be explained below, in some embodiments of the present invention, the rigid holder may comprise hinges or other mechanical elements allowing a motion of portions of the rigid holder relative to each other.

The connection element 112 can comprise a solenoid provided in the first portion 102 of the rigid holder 101 and a permanent magnet provided in the second portion of the rigid holder 101. If an electric current is flown through the solenoid, a magnetic field is created. The orientation of the magnetic field can be adapted such that the permanent magnet in the second portion 103 of the rigid holder 101 is attracted. Thus, the first portion 102 and the second portion 103 of the rigid holder 101 are connected with each other. In order to detach the second portion 103 from the first portion 102, the electric current can be turned off. Alternatively, the direction of the electric current can be reversed. Thus, the second portion 103 of the rigid holder 101 is pushed away from the first portion 102.

In other embodiments of the present invention, the permanent magnet can be replaced by a piece of ferromagnetic material. If a current is applied to the solenoid, the piece of ferromagnetic material is attracted by the solenoid. Alternatively, the second portion 103 of the rigid holder 101 can be manufactured from a ferromagnetic material such as stainless steel.

In other embodiments of the present invention, the first portion 102 and the second portion 103 of the rigid holder 101 can be connected by means of a bayonet coupling. In still further embodiments, one of the first portion 102 and the second portion 103 can comprise an external thread and the other can comprise a mating internal thread.

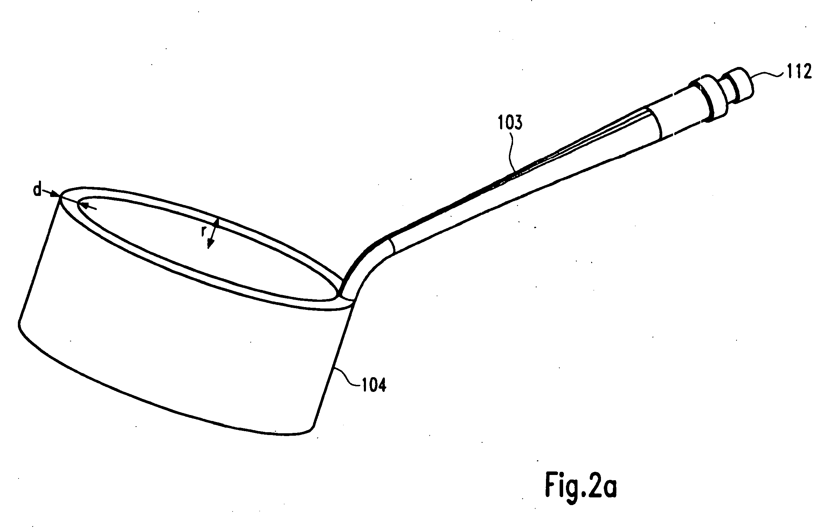

An inflatable element 104 is connected to the rigid holder 101. Fig. 1 shows the inflatable element in a non-inflated state. A schematic perspective view of the second portion 103 of the rigid holder 101 and the inflatable element 104 with the inflatable element 104 in its inflated state is shown in Fig. 2a.

The inflatable element 104 has a toroidal shape. In the following, the term "toroidal shape" shall generally denote any annularly closed configuration, irrespective of any deformation which, in particular, may occur if the inflatable element 104 is in its non-inflated state. In the embodiment shown in Figs. 1-3, the inflatable element in its non-inflated state assumes a folded configuration as shown in Fig. 1. In its inflated state, the inflatable element 104 assumes the shape of a cylindrical ring as shown in Figs. 2a to 2c. Advantageously, the folded configuration of the inflatable element in its non-inflated state simplifies an insertion of the heart valve retractor into a blood vessel such as the aorta or the atrium of the patient's heart through an incision.

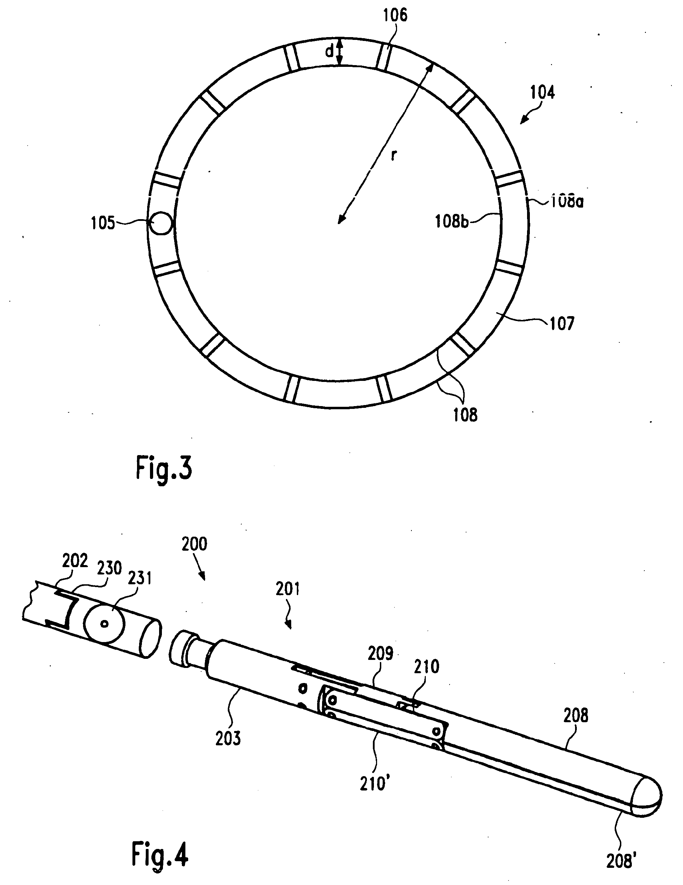

A schematic cross-sectional view of the inflatable element 104 in its inflated state is shown in Fig. 3.

The rigid holder 101 comprises a fluid line 105 adapted to supply fluid to the inflatable element. The fluid line 105 extends through the first portion 102 and the second portion 103 of the rigid holder 101, an end of the fluid line 105 being provided inside the inflatable element 104. At the connection element 112, a fluid tight connection between the portions of the fluid line 105 in the first and the second portion of the rigid holder 101 is established.

The second portion 103 of the rigid holder 101 comprises a valve closable on detachment of the second portion 103. While in some embodiments of the present invention, the valve is manually operable, in other embodiments, the valve can be adapted such that the valve is automatically closed when the second portion 103 of the rigid holder 101 is detached from the first portion. To this end, the valve can be a solenoid valve which is operable by an electric voltage supplied via the first portion 102. The solenoid valve can be configured such that it is in an open state as long as the electric voltage is applied and is switched to a closed state when the electric voltage is no longer applied as the first portion 102 and the second portion 103 of the rigid holder are separated from each other. Alternatively, the valve can be a mechanical valve.

The inflatable element 104 can comprise a material having an anisotropic elasticity. To this end, the inflatable element 104 is provided with an elastic envelope 108. In some embodiments of the present invention, the elastic envelope 108 can comprise silicone. The elastic envelope 108 encloses an inner volume 107 of the inflatable element 104 which is fillable with fluid via fluid line 105. A portion 108a of the elastic envelope 108 is provided at an outer circumferential surface of the toroidal inflatable element 104. Another portion 108b of the elastic envelope 108 is provided at an inner circumferential surface of the inflatable element 104. Spacer elements 106 which are provided as chordal bridges connect the portions 108a, 108b of the elastic envelope 108 with each other.

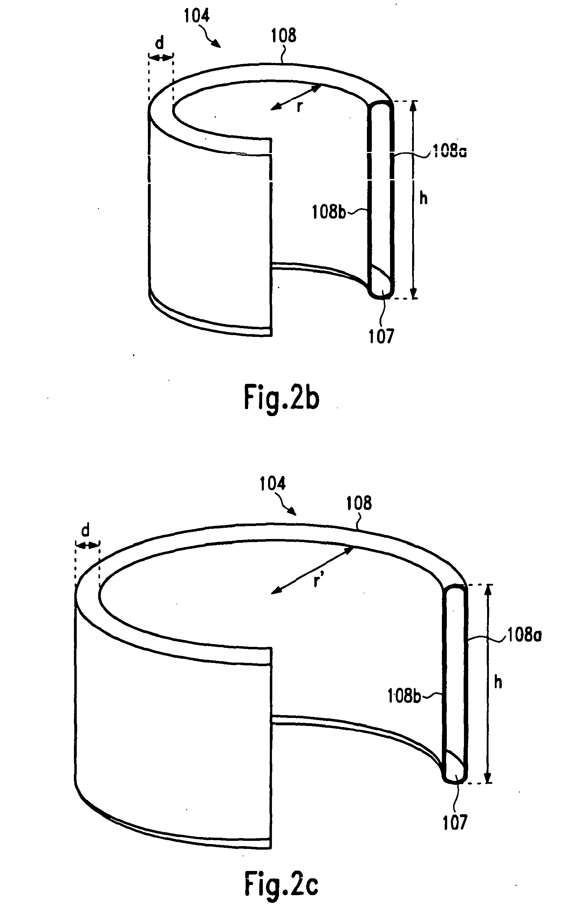

Figures 2b and 2c show schematic perspective views of the inflatable element 104, wherein a section of the inflatable element 104 and the rigid holder 103 have been omitted. Fig. 2b shows the inflatable element 104 being inflated to a first pressure and Fig. 2c shows the inflatable element 104 being inflated to a second pressure, wherein the second pressure is greater than the first pressure.

The spacer elements 106 are adapted to maintain a predetermined distance d between the portions 108a, 108b of the elastic envelope 108. To this end, the spacer elements can be made from a material having a lower elasticity than the material of the elastic envelope 108, or a high strength material, for example a aramid fiber material such as Kevlar^{®} which is conventionally used in parachutes.

If the inflatable element 104 is inflated, the spacer elements 106 substantially maintain the predetermined distance d between the inner and the outer circumferential surface of the inflatable element. Due to the elasticity of the elastic envelope 108, however, the radius r of the inflatable element 104 may vary, depending on the pressure of the fluid in the inner volume 107 of the inflatable element 104, wherein a greater pressure leads to a greater radius of the inflatable element 104.

The spacer elements 106 may further be adapted to maintain a predetermined height h of the inflatable element. Thus, when the pressure in the inflatable element 204 is increased from the first pressure to the second pressure, the radius r of the inflatable element may increase to a greater radius r, while the predetermined distance d between the inner and the outer circumferential surface of the inflatable element and the height h are maintained.

In the application of the heart valve retractor 100 in surgery, an opening is provided in a blood vessel of the patient. If surgery of the aortic valve is performed, the opening can be provided as a transversal incision of the aorta in the aortic root. Conversely, if surgery of the mitral valve is performed, the opening is provided as an incision in the left atrium of the patient's heart. Thereafter, the surgeon may grip the first portion 102 of the rigid holder and insert the inflatable portion 104 of the heart valve retractor 100 into the opening.

When the inflatable portion 104 is located inside the blood vessel, fluid is supplied to the inner volume 107 of the inflatable element 104 via the fluid line 105. The fluid can be a liquid, for example water, or a gas, for example air or carbon dioxide (CO₂). In the inflation, the inner volume 107 of the inflatable element 104 is filled with fluid. Thereby, the inflatable element 104 changes from the folded configuration shown in Fig. 1 to the ring-shaped configuration shown in Figs. 2a to 2c and the outer circumferential surface of the inflatable element 104 abuts the inner surface of the blood vessel. The pressure of the fluid supplied to the inflatable element can be controlled such that the radius r of the inflatable element conforms to the inner radius of the blood vessel. Thereby, the heart valve is exposed in a round manner for surgical manipulations. As a further effect, the coronary ostea are blocked. Thus, the flow of blood is inhibited, which allows for a clean surgical field.

After the inflation of the inflatable portion 104, the valve in the second portion 103 of the heart valve retractor is closed and the first portion 102 of the rigid holder is disconnected. Since the valve prevents a flow of the fluid out of the inner volume 107 of the inflatable element after the disconnection of the first portion 102 of the rigid holder 101, the inflatable element 104 remains inflated after the disconnection of the first portion 102. Thus, the inflatable element 104 remains inside the blood vessel, thus exposing the heart valve for surgical manipulations.

After the surgical manipulations of the heart valve have been performed, the inflatable element 104 is deflated and the heart valve retractor 100 is removed. The first portion 102 of the rigid holder 101 may be attached to the second portion 103 of the rigid holder 101 in order to deflate the inflatable element 104 and to remove the inflatable element 104 from the blood vessel.

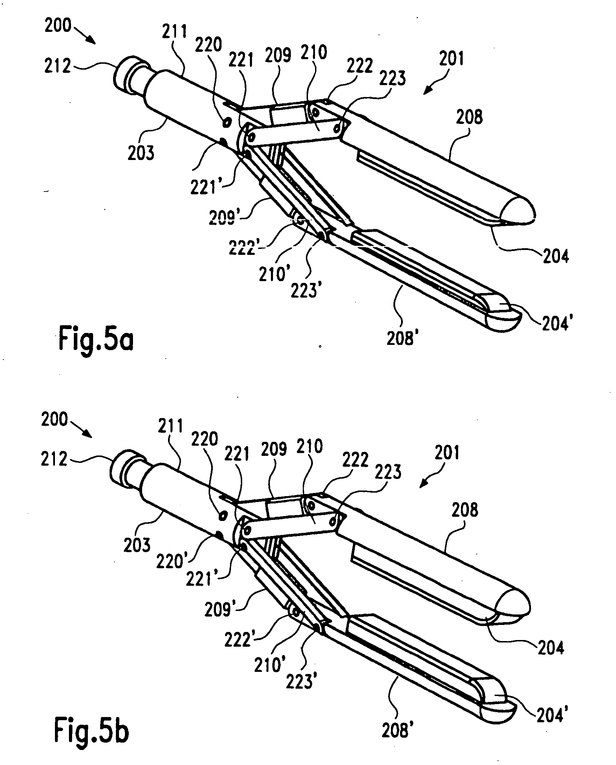

Further embodiments of the present invention will be described with reference to Figs. 4 and 5.

Fig. 4 shows a schematic perspective view of a blood vessel clamp 200 according to an embodiment of the present invention.

The blood vessel clamp 200 comprises a rigid holder 201 comprising a first portion 202 and a second portion 203. The first portion 202 and the second portion 203 are detachably connected with each other. In Fig. 4, the first portion 202 and the second portion 203 are shown in the disconnected state. The second portion 203 of the rigid holder 201 is movable between a consolidated configuration shown in Fig. 4 and an operation configuration shown in Figs. 5a and 5b. The second portion 203 of the rigid holder 201 comprises a first clamp portion 208 and a second clamp portion 208'. In the operation configuration, a blood vessel is insertable between the first clamp portion 208 and the second clamp portion 208'.

Arms 209, 210 connect the first clamp portion 208 with a shaft 211. Similarly, the second clamp portion 208' is connected to the shaft 211 via arms 209, 210. The arms 209, 210, 209', 210' pivotably engage the clamp portions 208, 208' and the shaft 211. To this end, bearings 220, 221, 222, 223, 220', 221', 222' and 223' can be provided.

The arms 209, 210, the shaft 211 and the first clamp portion 208 form a parallelogram. Hence, when the arms are pivoted, the first clamp portion will remain substantially parallel to the shaft 211 while moving away from a central axis of the shaft 211. Similarly, the shaft 211, the arms 209', 210' and the second clamp portion 208' form a parallelogram such that the second clamp portion 208' remains substantially parallel to the shaft when the arms are pivoted in order to move the second clamp portion 208' away from the central axis of the shaft 211.

In order to pivot the arms 209, 210, 209', 210', each of arms 209, 209' can comprise an extension extending beyond bearings 220 and 221 into the interior of the shaft 211. A linkage may be connected to each of the extensions. If the linkage is pushed in a direction towards the clamp portions 208, 208', the clamp portions are forced apart and the second portion 202 of the rigid holder 201 is moved into the operation configuration shown in Figs. 5a and 5b. Conversely, if the linkage is drawn in a direction towards the first portion 202 of the rigid holder 201, the clamp portions 208, 208' are moved towards each other and the second portion 202 of the rigid holder 201 is moved into the consolidated configuration shown in Fig. 4.

In some embodiments of the present invention, the linkage may be replaced by driving means of another type, for example by a spindle driven by a motor. The motor can be an electric motor.

The first portion 202 of the rigid holder 201 comprises a first hinge 230 and a second hinge 231. An axis of rotation of the first hinge 230 and an axis of rotation of the second hinge 231 may be oriented perpendicular to each other and to a central axis of the first portion 202 of the rigid holder 202. Thus, when the blood vessel clamp 200 is inserted into the patient's body, the second portion 203 of the rigid holder 201 may be moved towards the blood vessel to be clamped off by turning the first portion 202 at the hinges 230,231

On the first clamp portion 208, a first inflatable element 204 is provided. Similarly, a second inflatable element 204' is provided on the second clamp portion 208'. Fluid supply lines extend through the first portion 202 and the second portion 203 of the rigid holder 201 and are adapted to supply fluid to the inflatable elements 204, 204' and to discharge fluid from the inflatable elements 204, 204'. Thus, the inflatable element 204 may be inflated by supplying fluid to the inflatable elements 204, 204'. Moreover, the inflatable elements 204, 204' can be deflated by discharging fluid from the inflatable elements 204, 204'. While Fig. 5a shows the inflatable elements 204, 204' in a deflated state, Fig. 5b shows the inflatable elements 204, 204' in an inflated state.

Similar to the embodiment described above with reference to Figs. 1-3, a valve being closable upon detachment of the second portion 203 of the rigid holder 201 from the first portion 202 is provided in the second portion 203 in order to allow the inflatable elements 204, 204' to remain inflated when the first portion 202 is removed.

Each of the inflatable elements 204, 204' may comprise a material having an anisotropic elasticity. Similar to the embodiments described above with reference to Figs. 1-3, this may be achieved by forming the inflatable elements 204, 204' from an elastic envelope and providing one or more spacer elements in each inflatable element to maintain a predetermined distance between two portions of the elastic envelope.

The blood vessel clamp 200 is particularly useful in minimally invasive heart surgery, wherein the blood vessel clamp 200 is provided as aortic clamp in order to clamp off the patient's aorta after the patient has been connected to the heart-lung apparatus. In order to insert the blood vessel clamp 200, a minimally invasive aditus to the patient's thorax is provided by means of methods known to persons skilled in the art. Then, the blood vessel clamp 200 is inserted into the thorax, wherein the second portion 203 of the rigid holder 201 is in the consolidated configuration.

Inside the patient's body, the second portion 203 of the rigid holder 201 is moved from the consolidated configuration into the operation configuration wherein the first clamp portion 208 and the second clamp portion 208' are spaced apart from each other. Thereafter, the clamp portions are moved towards the patient's aorta such that the aorta is inserted between the clamp portions 208, 208'. Then, the clamp portions 208, 208' are moved towards each other such that the clamp portions 208, 208' rest against the aorta.

Fluid is supplied to inflatable elements 204, 204' via the fluid supply lines provided in the rigid holder 201 in order to inflate the inflatable elements 204, 204'. Due to the inflation of the inflatable elements 204, 204', the patient's aorta is compressed and the flow of blood through the aorta is inhibited. Thereafter, the first portion 202 of the rigid holder 201 may be detached from the second portion 203, the valve in the second portion 203 being closed upon the detachment of the first portion such that the inflatable portions 204, 204' remain inflated and the patient's aorta remains blocked.

After the completion of the surgery, the first portion 202 of the rigid holder 202 is attached to the second portion 203. Thereafter, the fluid is discharged from the inflatable elements 204, 204' to deflate the inflatable elements 204, 204'. Then, the blood vessel clamp 200 is removed from the patient's body.

## Claims

1. Surgical retractor (100, 200) comprising a rigid holder (101, 201 ), comprising at least one inflatable element (104, 204, 204' ) connected to said rigid holder (101, 201) and being adapted to exhibit a force on a part of a human or animal body upon inflation;
wherein said rigid holder (101, 201) comprises a fluid line (105) adapted to supply fluid to said at least one inflatable element (104, 204, 204') and/or to discharge fluid from said at least one inflatable element (104, 204, 204'); and
wherein said rigid holder (101, 201) comprises a first portion (102, 202) and a second portion (103, 203) detachably connected with each other, said second portion (103, 203) being connected to said inflatable element (104, 204, 204')
**characterized in that**
said fluid line extends through said first portion (102, 202) and said second portion (103, 203), said second portion (103, 203) comprising a valve closable on detachment of said first portion (102, 202).

2. Surgical retractor (100, 200) according to claim 1, wherein said at least one inflatable element 104, 204, 204') comprises an anisotropic elasticity.

3. Surgical retractor (100, 200) according to claim 2, wherein said at least one inflatable element (104, 204, 204') is extensible in a first dimension and non-extensible in a second dimension.

4. Surgical retractor (100, 200) according to claim 3, wherein said at least one inflatable element (104, 204, 204') comprises an elastic envelope (108) and at least one spacer element (106) adapted to maintain a predetermined distance between two portions (108a, 108b) of said elastic envelope (108).

5. Surgical retractor (100, 200) according to claim 4, wherein said two portions (108a, 108b) of said elastic envelope (108) are provided on opposite surfaces of an inner volume (107) of said inflatable element (104, 204, 204').

6. Surgical retractor (100, 200) according to claim 5, wherein said at least one spacer element (106) comprises a chordal bridge.

7. Surgical retractor (100) according to any of the preceding claims, wherein said surgical retractor (100) is configured as a heart valve retractor.

8. Surgical retractor (100) according to claim 7, wherein said at least one inflatable element (104) has a toroidal shape comprising an inner circumferential surface and an outer circumferential surface and wherein said inflatable element (104) comprises a plurality of spacer elements (106) provided inside said inflatable element (104) and being adapted to maintain a predetermined distance between said inner circumferential surface and said outer circumferential surface.

9. Surgical retractor (200) according to any of claims 1 to 6, wherein said surgical retractor (200) is configured as a blood vessel clamp.

10. Surgical retractor (200) according to claim 9, wherein said rigid holder (201) comprises a first clamp portion (208) and a second clamp portion (208'), a blood vessel being insertable between said first clamp portion (208) and said second clamp portion (208'), an inflatable element (204, 204') being provided on each of said first clamp portion (208) and said second clamp portion (208').

## Patentansprüche

1. Chirurgischer Retraktor (100, 200), der einen starren Halter (101, 201) umfasst, der wenigstens ein aufblasbares Element (104, 204, 204') umfasst, das mit dem starren Halter (101, 201) verbunden und so eingerichtet ist, dass es beim Aufblasen eine Kraft auf einen menschlichen oder tierischen Körper ausübt;
wobei der starre Halter (101, 201) eine Fluidleitung (105) umfasst, die so eingerichtet ist, dass sie dem wenigstens einen aufblasbaren Element (104, 204, 204') Fluid zuführt und/oder Fluid aus dem wenigstens aufblasbaren Element (104, 204, 204') ableitet; und
wobei der starre Halter (101, 201) einen ersten Abschnitt (102, 202) und einen zweiten Abschnitt (103, 203) umfasst, die lösbar miteinander verbunden sind, und der zweite Abschnitt (103, 203) mit dem aufblasbaren Element (104, 204, 204') verbunden ist,
**dadurch gekennzeichnet, dass**
die Fluidleitung durch den ersten Abschnitt (102, 202) und den zweiten Abschnitt (103, 203) hindurch verläuft und der zweite Abschnitt (103, 203) ein Ventil umfasst, das beim Lösen des ersten Abschnitts (102, 202) geschlossen werden kann.

2. Chirurgischer Retraktor (100, 200) nach Anspruch 1, wobei das wenigstens eine aufblasbare Element (104, 204, 204') eine anisotrope Elastizität aufweist.

3. Chirurgischer Retraktor (100, 200) nach Anspruch 2, wobei das wenigstens eine aufblasbare Element (104, 204, 204') in einer ersten Dimension ausgedehnt werden kann und in einer zweiten Dimension nicht ausgedehnt werden kann.

4. Chirurgischer Retraktor (100, 200) nach Anspruch 3, wobei das wenigstens eine aufblasbare Element (104, 204, 204') eine elastische Umhüllung (108) und wenigstens ein Abstandshalteelement (106) umfasst, das so eingerichtet ist, dass es einen vorgegebenen Abstand zwischen zwei Abschnitten (108a, 108b) der wenigstens einen elastischen Umhüllung (108) aufrechterhält.

5. Chirurgischer Retraktor (100, 200) nach Anspruch 4, wobei sich die zwei Abschnitte (108a, 108b) der elastischen Umhüllung (108) an einander gegenüberliegenden Flächen eines inneren Volumens (107) des aufblasbaren Elementes (104, 204, 204') befinden.

6. Chirurgischer Retraktor (100, 200) nach Anspruch 5, wobei das wenigstens eine Abstandshalteelement (106) eine Sehnenbrücke (chordal bridge) umfasst.

7. Chirurgischer Retraktor (100) nach einem der vorangehenden Ansprüche, wobei der chirurgische Retraktor (100) als ein Herzklappenretraktor ausgeführt ist.

8. Chirurgischer Retraktor (100) nach Anspruch 7, wobei das wenigstens eine aufblasbare Element (104) eine Ringform hat, die eine Innenumfangsfläche und eine Außenumfangsfläche umfasst, und das aufblasbare Element (104) eine Vielzahl von Abstandshalteelementen (106) umfasst, die sich im Inneren des aufblasbaren Elementes (104) befinden und so eingerichtet sind, dass sie einen vorgegebenen Abstand zwischen der Innenumfangsfläche und der Außenumfangsfläche aufrechterhalten.

9. Chirurgischer Retraktor (200) nach einem der Ansprüche 1 bis 6, wobei der chirurgische Retraktor (200) als eine Blutgefäßklemme ausgeführt ist.

10. Chirurgischer Retraktor (200) nach Anspruch 9, wobei der starre Halter (201) einen ersten Klemmenabschnitt (208) und einen zweiten Klemmenabschnitt (208') umfasst, ein Blutgefäß zwischen den ersten Klemmenabschnitt (208) und den zweiten Klemmenabschnitt (208') eingeführt werden kann und ein aufblasbares Element (204, 204') jeweils an dem ersten Klemmenabschnitt (208) und dem zweiten Klemmenabschnitt (208') vorhanden ist.

## Revendications

1. Ecarteur chirurgical (100, 200) comprenant un support rigide (101, 201) auquel est relié au moins un élément gonflable (104, 204, 204') apte à exercer une force sur une partie d'un corps humain ou animal, une fois gonflé ;
le support rigide (101, 201) comprenant un conduit pour fluide (105) apte à amener un fluide dans ledit élément gonflable (104, 204, 204') et/ou à évacuer un fluide de celui-ci ; et
le support rigide (101, 201) comprenant une première partie (102, 202) et une seconde partie (103, 203) reliées entre elles de manière amovible, la seconde partie (103, 203) étant reliée audit élément gonflable (104, 204, 204'),
**caractérisé en ce que** le conduit pour fluide traverse la première partie (102, 202) et la seconde partie (103, 203), la seconde partie (103, 203) comprenant une valve qui est apte à se fermer quand on enlève la première partie (102, 202).

2. Ecarteur chirurgical (100, 200) selon la revendication 1, dans lequel ledit élément gonflable (104, 204, 204') présente une élasticité anisotrope.

3. Ecarteur chirurgical (100, 200) selon la revendication 2, dans lequel ledit élément gonflable (104, 204, 204') est extensible dans une première dimension et non extensible dans une seconde dimension.

4. Ecarteur chirurgical (100, 200) selon la revendication 3, dans lequel ledit élément gonflable (104, 204, 204') comprend une enveloppe élastique (108) et au moins un élément d'écartement (106) qui est apte à maintenir une distance prédéterminée entre deux parties (108a, 108b) de l'enveloppe élastique (108).

5. Ecarteur chirurgical (100, 200) selon la revendication 4, dans lequel les deux parties (108a, 108b) de l'enveloppe élastique (108) sont prévues sur des surfaces opposées d'un volume intérieur (107) de l'élément gonflable (104, 204, 204').

6. Ecarteur chirurgical (100, 200) selon la revendication 5, dans lequel ledit élément d'écartement (106) comprend un pont à la corde.

7. Ecarteur chirurgical (100) selon l'une quelconque des revendications précédentes, qui est conçu comme un écarteur de valve cardiaque.

8. Ecarteur chirurgical (100) selon la revendication 7, dans lequel ledit élément gonflable (104) a une forme toroïdale qui comprend une surface circonférentielle intérieure et une surface circonférentielle extérieure, et dans lequel ledit élément gonflable (104) renferme plusieurs éléments d'écartement (106) qui sont aptes à maintenir une distance prédéterminée entre la surface circonférentielle intérieure et la surface circonférentielle extérieure.

9. Ecarteur chirurgical (200) selon l'une quelconque des revendications 1 à 6, qui est conçu comme un clamp pour vaisseau sanguin.

10. Ecarteur chirurgical (200) selon la revendication 9, dans lequel le support rigide (201) comprend une première partie de clamp (208) et une seconde partie de clamp (208'), un vaisseau sanguin pouvant être placé entre la première partie de clamp (208) et la seconde partie de clamp (208'), et un élément gonflable (204, 204') étant prévu sur la première partie de clamp (208) et sur la seconde partie de clamp (208').
